# EUROPÄISCHE PATENTANMELDUNG

(11) **EP 4 303 209 A1**
(43) Veröffentlichungstag der Anmeldung: **10.01.2024**
(21) Anmeldenummer: 22183349.4
(22) Anmeldetag: 06.07.2022
(51) Int. Cl.: C07C 45/50, C07C 47/32, C07C 29/141, C07C 31/27

(54) **VERFAHREN ZUR HERSTELLUNG DES DIALDEHYDS VON VINYLCYCLOHEXEN**

(71) Anmelder: Evonik Oxeno GmbH & Co. KG, 45772 Marl (DE)
(72) Erfinder: FRANKE, Robert, 45772 Marl (DE); SCHNEIDER, Carolin, 40789 Monheim am Rhein (DE); JACKSTELL, Ralf, 18106 Rostock (DE); BELLER, Matthias, 18211 Ostseebad Nienhagen (DE)
(74) Vertreter: Evonik Patent Association

(57) **Zusammenfassung**

Verfahren zur Herstellung des Dialdehyds von Vinylcyclohexen.

## Beschreibung

Die vorliegende Erfindung betrifft ein Verfahren zur Herstellung des Dialdehyds von Vinylcyclohexen.

In US 2009/0171125 A1 wird ein Verfahren zur Hydroformylierung von cyclischen Olefinen beschrieben. Hierbei kommt ein Rh-Katalysator zum Einsatz.

Der vorliegenden Erfindung lag die Aufgabe zugrunde, ein neues Hydroformylierungsverfahren bereitzustellen. Das Verfahren soll hierbei eine gegenüber der aus dem Stand der Technik bekannten Methode gesteigerte Ausbeute liefern.

Diese Aufgabe wird gelöst durch ein Verfahren gemäß Anspruch 1.

Verfahren umfassend die Verfahrensschritte:
a) Vorlegen von Vinylcyclohexen;
b) Zugabe einer Verbindung gemäß der Formel (**I**): 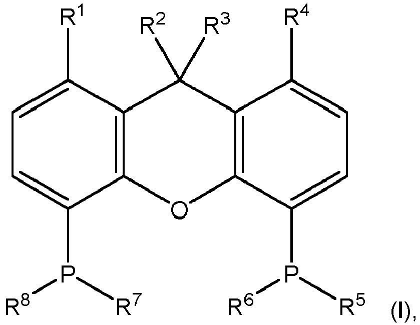 wobei R¹, R², R³, R⁴, R⁵, R⁶, R⁷, R⁸ ausgewählt sind aus: -H, -(C₁-C₁₂)-Alkyl, -Ph;
c) Zugabe einer Pt-Verbindung, welche zur Ausbildung eines Komplexes befähigt ist;
d) Zugabe einer Iod-Verbindung;
e) Zuführen von CO und H₂;
f) Erwärmen des Reaktionsgemisches aus a) bis e), wobei das Vinylcyclohexen zum Dialdehyd umgesetzt wird.

Hierbei können die Verfahrensschritte a) bis e) in beliebiger Reihenfolge erfolgen. Üblicherweise erfolgt die Zugabe von CO und H₂ jedoch, nachdem die Reaktionspartner in den Schritten a) bis d) vorgelegt wurden.

Hierbei können die Verfahrensschritte c) und d) auch in einem Schritt, beispielsweise durch Zugabe von Ptl₂, erfolgen.

In einer Variante des Verfahrens erfolgt die Zugabe der Pt-Verbindung und der Iod-Verbindung in einem Schritt, durch Zugabe von Ptl₂.

Der Begriff (C₁-C₁₂)-Alkyl umfasst geradkettige und verzweigte Alkylgruppen mit 1 bis 12 Kohlenstoffatomen. Vorzugsweise handelt es sich dabei um (C₁-C₈)-Alkylgruppen, besonders bevorzugt (C₁-C₆)-Alkyl, am meisten bevorzugt (C₁-C₄)-Alkyl.

Geeignete (C₁-C₁₂)-Alkylgruppen sind insbesondere Methyl, Ethyl, Propyl, Isopropyl, n-Butyl, iso-Butyl, sec-Butyl, tert-Butyl, n-Pentyl, 2-Pentyl, 2-Methylbutyl, 3-Methylbutyl, 1,2-Dimethylpropyl, 1,1-Dimethylpropyl, 2,2-Dimethylpropyl, 1-Ethylpropyl, n-Hexyl, 2-Hexyl, 2-Methylpentyl, 3-Methylpentyl, 4-Methylpentyl, 1,1-Dimethylbutyl, 1,2-Dimethylbutyl, 2,2-Dimethylbutyl, 1,3-Dimethylbutyl, 2,3-Dimethylbutyl, 3,3-Dimethylbutyl, 1,1,2-Trimethylpropyl, 1,2,2-Trimethylpropyl, 1-Ethylbutyl, 1-Ethyl-2-methylpropyl, n-Heptyl, 2-Heptyl, 3-Heptyl, 2-Ethylpentyl, 1-Propylbutyl, n-Octyl, 2-Ethylhexyl, 2-Propylheptyl, Nonyl, Decyl.

In einer Variante des Verfahrens stehen R¹ und R⁴ für -H.

In einer Variante des Verfahrens stehen R⁵, R⁶, R⁷, R⁸ für -Ph.

In einer Variante des Verfahrens stehen R² und R³ für -(C₁-C₁₂)-Alkyl.

In einer Variante des Verfahrens stehen R² und R³ für -CH₃.

In einer Variante des Verfahrens weisen die Verbindung (**I**) die Struktur (**1**) auf:

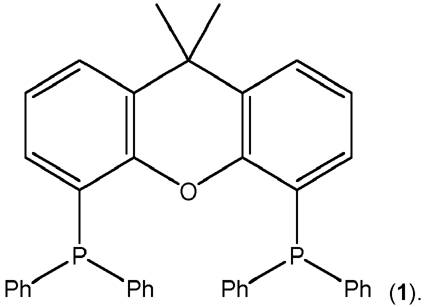

In einer Variante des Verfahrens ist die Pt-Verbindung ausgewählt aus: Pt(II)I₂, Pt(IV)I₄, Diphenyl(1,5-COD)Pt(II), Pt(II)(acac)₂, Pt(0)(PPh₃)₄, Pt(0)(DVTS)-Lösung (CAS: 68478-92-2), Pt(0)(Ethylen)(PPh₃)₂, Tris(benzylidenaceton)Pt(0), Pt(II)(OAC)₂-Lösung, Pt(0)(t-Bu)₂, Pt(II)(COD)Me₂, Pt(II)(COD)I₂, Pt(IV)IMe₃, Pt(II)(Hexafluoroacetylacetonat)₂

In einer Variante des Verfahrens ist die Pt-Verbindung ausgewählt aus: Pt(II)I₂, Pt(II)(acac)₂.

In einer Variante des Verfahrens ist die Pt-Verbindung Pt(II)I₂.

In einer Variante des Verfahrens ist die Iod-Verbindung ausgewählt aus: Alkalihalogenid, Erdalkalihalogenid, NH₄X, Alkylammoniumhalogenid, Dialkylhalogenid, Trialkylhalogenid, Tetraalkylhalogenid, Cycloalkylammoniumhalogenid.

In einer Variante des Verfahrens ist die Iod-Verbindung ausgewählt aus: Pt(II)I₂, Lil.

In einer Variante des Verfahrens wird Ptl₂ in einer Menge zugegeben, gemessen in mol% bezogen auf Vinylcyclohexen, so dass der Wert im Bereich von 0,1 mol% bis 5 mol% liegt.

In einer Variante des Verfahrens wird Ptl₂ in einer Menge zugegeben, gemessen in mol% bezogen auf Vinylcyclohexen, so dass der Wert im Bereich von 0,1 mol% bis 3 mol% liegt.

In einer Variante des Verfahrens wird Ptl₂ in einer Menge zugegeben, gemessen in mol% bezogen auf Vinylcyclohexen, so dass der Wert im Bereich von 0,1 mol% bis 1 mol% liegt.

In einer Variante des Verfahrens umfasst dieses den zusätzlichen Verfahrensschritt e'): e') Zugabe eines Lösungsmittels.

In einer Variante des Verfahrens ist das Lösungsmittel ausgewählt aus: THF, DCM, ACN, Heptan, DMF, Toluol, Texanol, Pentan, Hexan, Octan, Isooctan, Decan, Dodecan, Cyclohexan, Benzen, Xylene, Marlotherm, Propylencarbonat, MTBE, Diglyme, Triglyme, Diethylether, Dioxan, Isopropanol, Tertbutanol, Isononanol, Isobutanol, Isopentanol, Ethylacetat.

In einer Variante des Verfahrens ist das Lösungsmittel ausgewählt aus: THF, DCM, ACN, Heptan, DMF, Toluol, Texanol.

In einer Variante des Verfahrens erfolgt das Zuführen von CO und H₂ bei einem Druck in einem Bereich von 1 MPa (10 bar) bis 6 MPa (60 bar).

In einer Variante des Verfahrens erfolgt das Zuführen von CO und H₂ bei einem Druck in einem Bereich von 1 MPa (20 bar) bis 6 MPa (50 bar).

In einer Variante des Verfahrens erfolgt das Erwärmen auf eine Temperatur im Bereich von 30 °C bis 150 °C.

In einer Variante des Verfahrens erfolgt das Erwärmen auf eine Temperatur im Bereich von 80 °C bis 140 °C.

In einer Variante des Verfahrens umfasst das Verfahren den zusätzlichen Verfahrensschritt g):
g) Umsetzen des Dialdehyds zum Diol.

In einer Variante des Verfahrens erfolgt die Umsetzung des Dialdehyds zum Diol unter Einsatz von "Shvos catalyst" (CAS 104439-77-2).

Neben dem Verfahren werden auch das Aldehydgemisch, (2a) und (2b), sowie das Alkoholgemisch (**3a**) und (**3b**) beansprucht.

Aldehydgemisch, umfassend die Verbindungen (**2a**) und (**2b**):

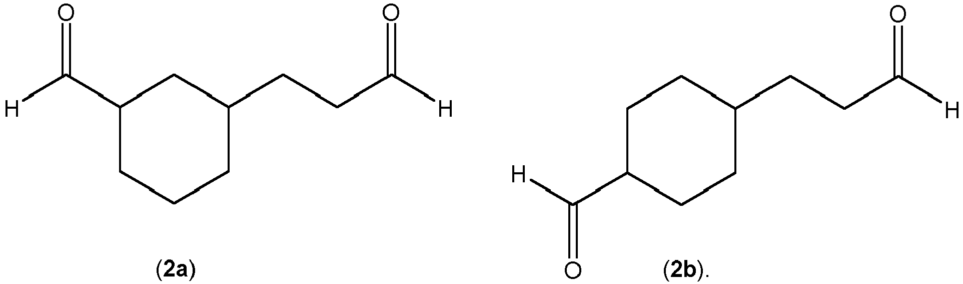

Alkoholgemisch, umfassend die Verbindungen (**3a**) und (**3b**):

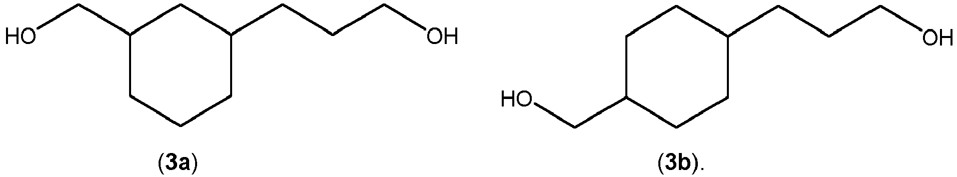

Im Folgenden soll die Erfindung anhand eines Ausführungsbeispiels näher erläutert werden.

### Versuchsbeschreibung

### Umsetzung von Vinylcyclohexen zum Dialdehyd

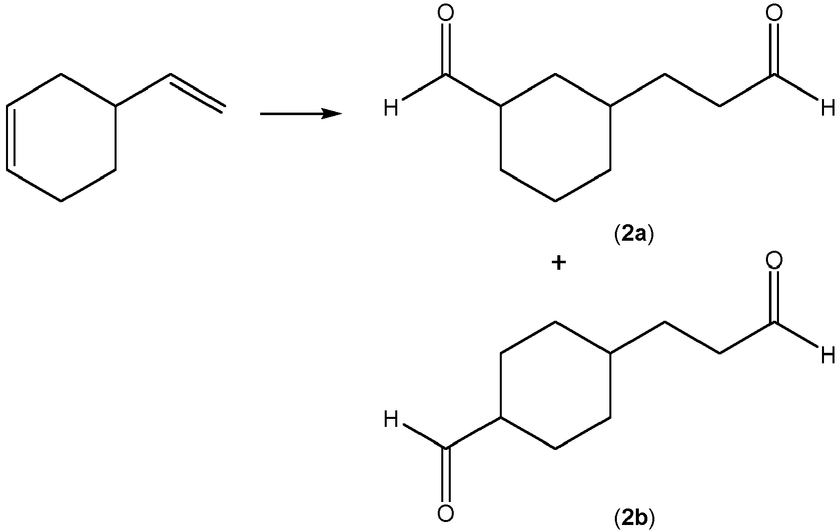

10 mmol 4-Vinylcyclohex-1-en, 10 ml absolutes Toluol, 0,5 mol% Ptl₂, 2,2 Äquivalente Xantphos (**1**) (bezogen auf Pt) werden unter Argon in einen 25 ml Stahlautoklav der Firma Parr Instruments gegeben. Es werden 40 bar Synthesegas (CO/H₂ = 1:1) aufgepresst und die Reaktion durch Aufheizen auf 120 °C und Rührung gestartet. Diese Reaktion wird für 3,5 h bei 40 bar / 120 °C durchgeführt. Anschließend wird der Autoklav abgekühlt, der Druckabgelassen und eine GC Probe genommen.

In einem Vergleichsversuch wurden anstelle von Ptl₂ Rh(acac)(CO)₂ hinzugegeben. Die Reaktion mit Rh(acac)(CO)₂ lief über 11 h.

Ausbeute Dialdehyd (**2a**) + (**2b**):
Ptl₂: 92%
Rh(acac)(CO)₂: < 14 %

### Umsetzung von Dialdehyd zum Diol

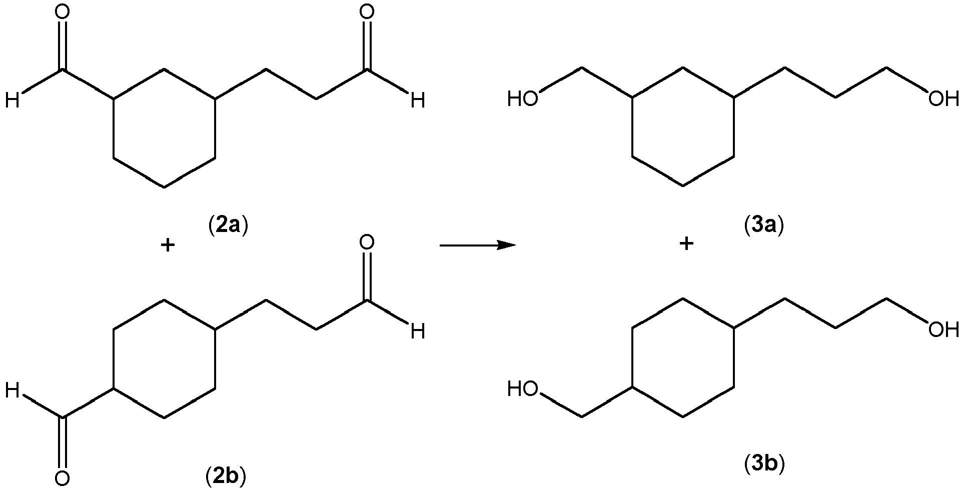

30 mmol der Isomerenmischung des Dialdehyds, 25 ml absolutes Toluol, 176 mg "Shvos catalyst" (CAS 104439-77-2) werden unter Argon in einen 100 ml Parr-Druckautoklaven überführt. Es werden 50 bar Wasserstoff aufgepresst und unter Rührung die Reaktion bei 100 °C für 1 h und für weitere 30 min bei 110 °C durchgeführt. Dann wird die Reaktion abgebrochen (Autoklav abgekühlt und der Druck abgelassen). Die Reaktionslösung wird in ein Schlenkgefäß überführt. Es bilden sich zwei Phasen, die untere Phase wird isoliert und im Vakuum von Toluol befreit. Es handelt sich um die Isomerenmischung des Diols (**3a**) + (**3b**).

Ausbeute Diol (**3a**) + (**3b**): 85 %

Wie die Versuchsergebnisse zeigen, wird die Aufgabe durch das erfindungsgemäße Verfahren gelöst.

## Patentansprüche

1. Verfahren umfassend die Verfahrensschritte:
a) Vorlegen von Vinylcyclohexen;
b) Zugabe einer Verbindung gemäß der Formel (**I**): 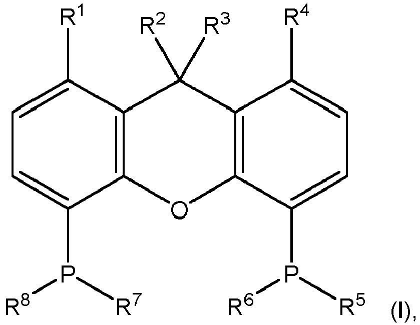 wobei R¹, R², R³, R⁴, R⁵, R⁶, R⁷, R⁸ ausgewählt sind aus: -H, -(C₁-C₁₂)-Alkyl, -Ph;
c) Zugabe einer Pt-Verbindung, welche zur Ausbildung eines Komplexes befähigt ist;
d) Zugabe einer Iod-Verbindung;
e) Zuführen von CO und H₂;
f) Erwärmen des Reaktionsgemisches aus a) bis e), wobei das Vinylcyclohexen zum Dialdehyd umgesetzt wird.

2. Verfahren nach Anspruch 1,
wobei R¹ und R⁴ für -H stehen.

3. Verfahren nach einem der Ansprüche 1 oder 2,
wobei R⁵, R⁶, R⁷, R⁸ für -Ph stehen.

4. Verfahren nach einem der Ansprüche 1 bis 3,
wobei R² und R³ für -(C₁-C₁₂)-Alkyl stehen.

5. Verfahren nach einem der Ansprüche 1 bis 4,
wobei R² und R³ für -CH₃ stehen.

6. Verfahren nach einem der Ansprüche 1 bis 5,
wobei die Verbindung (**I**) die Struktur (**1**) aufweist:

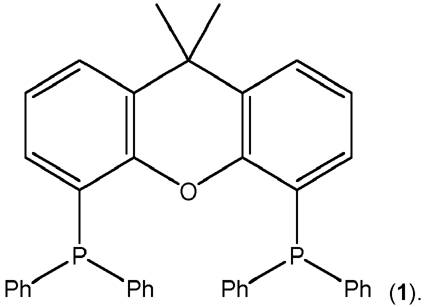

7. Verfahren nach einem der Ansprüche 1 bis 6,
wobei die Pt-Verbindung ausgewählt ist aus: Pt(II)I₂, Pt(IV)I₄, Diphenyl(1,5-COD)Pt(II), Pt(II)(acac)₂, Pt(0)(PPh₃)₄, Pt(0)(DVTS)-Lösung (CAS: 68478-92-2), Pt(0)(Ethylen)(PPh₃)₂, Tris(benzylidenaceton)Pt(0), Pt(II)(OAC)₂-Lösung, Pt(0)(t-Bu)₂, Pt(II)(COD)Me₂, Pt(II)(COD)I₂, Pt(IV)IMe₃, Pt(II)(Hexafluoroacetylacetonat)₂.

8. Verfahren nach einem der Ansprüche 1 bis 7,
wobei Ptl₂ in einer Menge zugegeben wird, gemessen in mol% bezogen auf Vinylcyclohexen, so dass der Wert im Bereich von 0,1 mol% bis 5 mol% liegt.

9. Verfahren nach einem der Ansprüche 1 bis 8,
umfassend den zusätzlichen Verfahrensschritt e'):
e') Zugabe eines Lösungsmittels.

10. Verfahren nach einem der Ansprüche 1 bis 9,
wobei das Verfahren den zusätzlichen Verfahrensschritt g) umfasst:
g) Umsetzen des Dialdehyds zum Diol.

11. Verfahren nach Anspruch 10,
wobei die Umsetzung des Dialdehyds zum Diol unter Einsatz von "Shvos catalyst" (CAS 104439-77-2) erfolgt.

12. Aldehydgemisch, umfassend die Verbindungen (**2a**) und (**2b**):

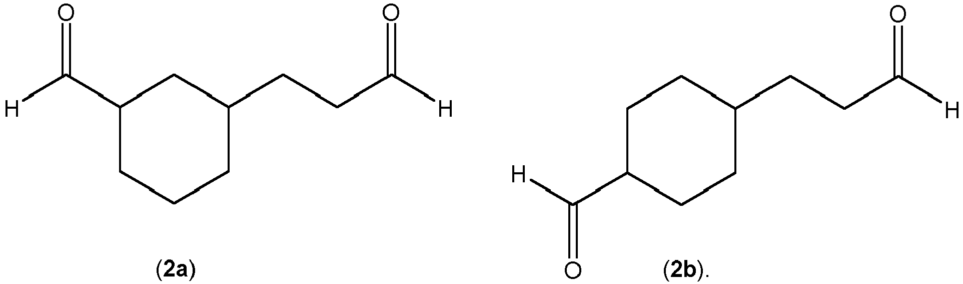

13. Alkoholgemisch, umfassend die Verbindungen (**3a**) und (**3b**):
